# EUROPÄISCHE PATENTANMELDUNG

(11) **EP 3 502 094 A1**
(43) Veröffentlichungstag der Anmeldung: **26.06.2019**
(21) Anmeldenummer: 17209218.1
(22) Anmeldetag: 21.12.2017
(51) Int. Cl.: C07C 329/00, C08F 293/00

(54) **VERFAHREN ZUR HERSTELLUNG VON TELECHELEN POLYOLEN AUS TRITHIOCARBONATEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung beschreibt H-funktionelle Trithiocarbonate als Reagenzien für die reversible Additions-Fragmentierungs-Kettenübertragungs-Polymerisation (RAFT) und deren Herstellung. Ein weiterer Gegenstand ist ein Verfahren zur Herstellung eines telechelen Polyols, bevorzugt telechelen Diols, mit einem doppelbindungshaltigen Monomer in Gegenwart eines Radikalstarters sowie telechele Polyole erhältlich nach diesem Verfahren.

## Beschreibung

Die vorliegende Erfindung beschreibt H-funktionelle Trithiocarbonate als Reagenzien für die reversible Additions-Fragmentierungs-Kettenübertragungs-Polymerisation und deren Herstellung. Ein weiterer Gegenstand ist ein Verfahren zur Herstellung eines telechelen Polyols, bevorzugt telechelen Diols, mit einem doppelbindungshaltigen Monomer in Gegenwart eines Radikalstarters sowie telechele Polyole erhältlich nach diesem Verfahren.

Unter Telechelen bzw. telechelen Oligomeren und telechelen Polymere werden im allgemeinen lineare Oligomere oder lineare Polymere mit niedrigen bis mittleren Molgewichten verstanden, die definierte funktionelle Gruppen an beiden Kettenenden aufweisen und somit über eine strikte Endgruppenfunktionalität verfügt. Es wird ebenfalls von funktionell terminierten oder α-ω funktionalisierten bzw. terminierten Molekülen gesprochen. Moleküle mit mehr als 2 definierten Endgruppenfunktionalitäten sind ebenfalls möglich. Diese werden bezüglich ihrer strikten Endgruppen pro Kettenende bezeichnet. Die Endgruppenfunktionalität, welche in der Folge auch als Funktionalität bezeichnet wird, ist als funktionelle Gruppe pro Kettenende definiert und wird mit dem Buchstaben F abgekürzt.

Telechele mit F ≥ 2 finden unter anderem Verwendung als Bausteine in Polyadditionsreaktionen wie z.B. zur Herstellung von Polyurethanen (PU) und Polyharnstoffen (Polyurea), in Polykondensationen wie z.B. zur Herstellung von Polyestern, Polycarbonaten, Polyamiden, und speziellen Polyethern, in Ringöffungspolymerisationen mit cyclischen Monomeren wie z.B. cyclischen Estern, cyclischen Carbonaten, cyclischen Ethern, cyclischen Säureanhydriden, Acetalen, Lacatmen usw. sowie als Abruchreagenzien mit gewünschten chemischen Funktionalitäten für andere polymerbildende Reaktionen. Des Weiteren finden spezielle Telechele Verwendung als Startern bzw. Kettentransferreagenzien mit Zerewitinow-aktivem Wasserstoff. Von höchster technischer Wichtigkeit sind dabei hydroxy terminierte Strukturen (siehe z.B. D. Dieterich et al., Prog. Org. Coatings, 1981, 9, 281). Dies gilt für (α-ω)-dihydroxy Telechele (F = 2) als auch für Strukturen mit F ≥ 2.
Polyvinyl- und Polyacrylverbindungen sind aus technisch-industrieller besonders interessant, da sie über eine große strukturelle Vielfalt verfügen, breite und / oder maßgeschneiderte chemischphysikalische Eigenschaften aufweisen und sich aus günstigen und großtonnagig verfügbaren Monomeren herstellen lassen.

Die selektive und effiziente Darstellung von oligomeren und polymeren Polyvinyl- und Polyacrylverbindungen mit definierten Molmassen und Molmassenverteilungen kann z.B. über lebende/kontrollierte radikalische Polymerisationen und mechanistisch verwandte Polymerisationen erfolgen. Kontrollierte freie radikalische Polymerisationen (engl. kurz CFRP), lebende freie radikalische Polymerisation (engl. kurz LFRP), stabile freie radikalische Polymerisationen (engl. SFRP), Atom Transfer Radikal Polymerisationen (kurz ATRP), reversible Additions-Fragmentierungs-Kettenübertragungs-Polymerisation (engl. kurz RAFT) und verwandte Methoden sind Stand der Technik zur Darstellung definierter Oligo- und Polymeren. Aber auch andere zumeist lebende Polymerisationen wie z.B. die Gruppentransfer Polymerisation (kurz GTP) udgl. sind als Stand der Technik zur Darstellung von definierter Oligo- und Polymeren gut beschrieben.

Die Darstellung von α-ω - Polymethacrylatdiolen durch anionische Polymerisation wie in Macromolecules, 1981, 14, 1599-1601 beschrieben gelingt nur unvollständig, die OH-Funktionalität beträgt lediglich F = 1,9.

Die Darstellung von α-ω - Polymethacrylatdiolen mittels GTP ist in EP0068887 (A1) und J. Polym. Sci., Polym. Lett. Ed., 1983, 21, 927-931 als mehrstufiger Prozess unter Verwendung von Ketensilylacetal beschrieben. Die funktionellen OH-Endgruppen werden erst durch Entschützen, d.h. Umwandlung der Silylgruppen, gebildet. Zum einen sind hohe Reinheiten der Monomere und Lösungsmittel nötig, zum anderen sind Preise und technische Verfügbarkeit der Silan-Initiatoren nachteilig. Auch die strikte Limitierung auf acrylische Monomere und der Bedarf an zusätzlichem Katalysator machen dieses Verfahren zur Synthese von Telechelen unattraktiv. Eine technische Anwendung bzw. Anwendbarkeit ist nicht gegeben.
Die Darstellung von α-ω - Polymethacrylatdiolen durch den Einsatz der Klasse der Iniferter-Substanzen (abgeleitet von der englischen Bezeichnung initiator - transfer agent - chain terminator) ist ebenfalls in J. Polymer Sci. Part A: Polymer Chem., 1989, 27, 1795-1809 beschrieben. Nachteilig an dieser Art von Verfahren ist zum einen die Tatsache, dass die Iniferter-Substanzen lichtinstabil sind und daher die Reaktion im Dunklen durchgeführt werden muss. Zudem lassen sich selbst bei hoher Reaktionsdauer nur Teilumsätze an Monomer erzielen und die Produktpolymere weisen sehr hohe Polydispersitäten (z.B. 2,63) auf.
Die Darstellung von α-ω - Polymethacrylatdiolen mittels Endgruppenumesterung wird in u.a. in Farbe & Lacke, 1999, 105, 24-29 beschrieben. Nachteilig an dieser Art von Verfahren ist zum einen die Zweistufigkeit der Synthese. Zum anderen ist die Methode lediglich auf Polyacrylate jedoch nicht auf Polyvinyle wie Polystyrole und Polyacrylnitrile übertragbar. Zusätzlich zu diesen Nachteilen sind die Polydispersitäten der Produkte mit 1,5 bis 1,8 hoch.

Die Darstellung von α-ω - Polymethacrylatdiolen und α-ω - Polyvinyldiolen mittels Atom Transfer Radikal Polymerisationen (kurz ATRP) mit DIT (engl. degenerative iodine transfer)-Technik wird in WO9920659(A1) beschrieben. Nachteilig an dieser Art von Verfahren ist zum einen die Zweistufigkeit der Synthese als auch die sehr anspruchsvollen Reaktionsbedingungen. Die Iodo-Zwischenstufen in der Polymerisation sind sehr anfällig gegen Oxidationen und diese Oxidationen führen zu ungewollten irreversiblen Kettenabbrüchen. Auch entstehen Salze als Abfallstoffe und es müssen verschiedene entgaste Lösungsmittel eingesetzt werden.

Die Darstellung von α-ω - Polymethacrylatdiolen mittels Atom Transfer Radikal Polymerisationen (kurz ATRP) wird in EP 0945469, US 6455645B und EP 1083186 beschrieben. Die hier beschriebenen Verfahren weisen den Nachteil auf, dass nach der eigentlichen Polymerreaktion weitere Schritte zwingend nötig sind. Zum einen die Umwandlung der Halogenidgruppen in die gewünschte funktionelle Gruppe ohne dabei andere funktionelle Gruppe, wie z.B. die der Monomere, zu verändern. Zum anderen müssen die gewonnenen Produkte aufwendig und quantitativ von Katalysatorspuren, wie z.B. Kupfer-Spezies, Bipyridinen, etc., gereinigt werden. Rückstände dieser Art beeinträchtigen bei nicht vollständiger Entfernung nicht nur die Färbung, sondern auch das Gebrauchsverhalten und die Oxidationsbeständigkeit der Produkte negativ.
Die Darstellung von α-ω - Polymethacrylatdiolen und α-ω - Polyvinyldiolen mittels lebender Polymerisation mittels Alkoxyaminen wird in EP 1083186, US 2003/0208021A1 und J. Am. Chem. Soc., 1994, 116, 11185 beschrieben. Nachteilig an dieser Methode ist, dass das Endcapping der erhaltenen Polymere reversible ist und zu "lebenden" Kettenende, d.h. einen chemisch noch in Teilen aktiven Endprodukt, welche ungewollte Reaktionen eingehen können, führt, sofern keine Abbruchreagenzien zusätzlich eingeführt werden.

Die Darstellung von dihydroxy-(α-ω)-terminierten telechelen Polymere mittels reversible Additions-Fragmentierungs-Kettenübertragungs-Polymerisation (RAFT-Polymerisation) unter Verwendung von funktionalisierten Trithiocarbonaten als Kettentransferreagenzien ist in Polymer, 2004, 45, 4413-4421 und Macromolecules, 2004, 37, 9761-9767 beschrieben. Jedoch ist die Darstellung des verwendeten Kettentransferreagenz S,S'-bis(2-hydroxylethyl-2'-butyrate)trithiocarbonat (Fig. 1) unter Verwendung einer sehr aufwendigen Festphasensynthese beschrieben. Die Konditionierung der Festphase dauert alleine 12 h, anschließend folgt eine längliche Trocknung sowie zusätzliche 12 h Reaktionszeit. Es werden lediglich 27 bzw. 48% Ausbeute an S,S'-bis(2-hydroxylethyl-2'-butyrate)trithiocarbonat erzielt. Darüber hinaus muss noch zusätzlich 2-Hydroxylethyl 2'-bromobutyrat als Vorstufe aufwendig synthetisiert werden. Dabei wird Benzol als karzinogenes und giftiges Lösungsmittel eingesetzt (vgl. J. Polym. Sci. A Polymer Chem., 2000, 38, 436-443). Zudem wird in Macromolecules, 2004, 37, 9761-9767 mittels ringöffenender Polymerisation mit Lactid das Kettentransferreagenz in einem weiteren Syntheseschritt modifiziert, bevor es zur Umsetzung mit ungesättigten Monomeren - der eigentlichen RAFT-Polymerisation - gebracht wird. Dieser Schritt reduziert den Gewichtsanteil welcher aus ungesättigten Monomeren stammt erheblich. 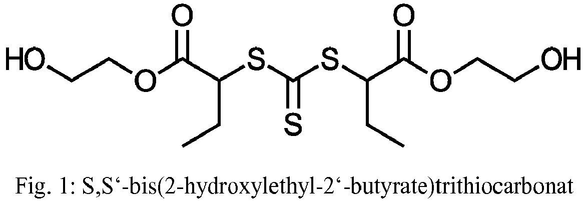 Die beschriebenen Umsetzungen mit vinylischen Monomeren zeigen Teilumsätze. Selbst die Massepolymerisation mit Styrol liefert nur bis zu 64% Umsatz nach 22 h Reaktionszeit. Insgesamt ist somit das beschrieben Verfahren zur Darstellung von dihydroxy-(α-ω)-terminierten telechelen Polymere aus technisch-industrieller Sicht ungeeignet.

Die Darstellung von α-ω-Polymethacrylatdiole und α-ω-Polystyroldiolen mittels reversible Additions-Fragmentierungs-Kettenübertragungs-Polymerisation (RAFT) unter Verwendung von funktionalisierten Trithiocarbonaten als Kettentransferreagenzien ist auch von T. Endo und Mitarbeitern in J. Polym. Sci. A Polymer Chem., 2013, 51, 318-326 bzw. in JP2014-210738A beschrieben. Dabei finden Trithiocarbonatdiole mit benzylischer Substitution der Trithiocarbonateinheiten als RAFT-Reagenzien Anwendung. Diese RAFT-Reagenzien sind nur über aufwendige Synthesen zugänglich und somit teuer in der Herstellung. Zudem zeichnen sich diese Strukturen durch verhältnismäßig hohe Molgewichte aus, was sich negativ auf die Strukturen und Eigenschaften der resultierenden RAFT-Produkte, α-ω-Diole, auswirkt. Insbesondere dann wenn - wie hier gezeigt - niedrige Molgewichte der RAFT-Produkte synthetisiert werden (z.B. mit Mₙ 1300 bis 5300 g/mol). Ein weiterer Nachteil der hier gezeigten Trithiocarbonatdiol RAFT-Reagenzien ist deren schlechte Reaktivität, die sich in niedrigen Umsatzraten und bei langen Reaktionszeiten (≥ 24 h) unvollständigen Monomerumsätzen zeigen. Somit ist dieses beschriebene Verfahren aus technisch-industrieller Sicht ungeeignet. Die Aufgabe der vorliegenden Erfindung bestand in der Bereitstellung eines einfachen und effizienten Verfahrens zur Herstellung von Reagenzien für die reversible Additions-Fragmentierungs-Kettenübertragungs-Polymerisation (RAFT Reagenzien) über möglichst wenige Verfahrensstufen und hoher Selektivität unter Verwendung von kommerziell gut verfügbaren Ausgangsstoffen im Vergleich zu dem im Stand der Technik offenbarten Verfahren.

Hierbei soll das RAFT Reagenz sich für die Additions-Fragmentierungs-Kettenübertragungs-Polymerisation von verschiedenen vinylischen und/oder acrylischen Monomeren eignen, wobei die resultierenden telechelen Polyols in einer hohen Ausbeute verbunden mit einer engen Molekulargewichtsverteilung des Techeles in moderater Reaktionszeit gebildet werden sollen.

Erfindungsgemäß wurde diese Aufgabe gelöst durch ein H-funktionelles Trithiocarbonat (A) als Reagenz für die reversible Additions-Fragmentierungs-Kettenübertragungs-Polymerisation (RAFT), wobei das H-funktionelle Trithiocarbonat (A) die Struktur (A-1) 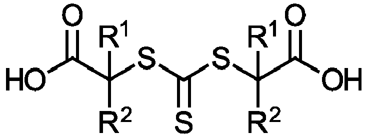 mit
- R¹: ausgewählt wird aus der Gruppe bestehend aus Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
- R²: ausgewählt wird aus der Gruppe bestehend aus Wasserstoff, Methyl, Ethyl, Propyl , iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl Octyl, Phenyl, Benzyl und Cyclohexyl
und/oder Struktur (A-2) 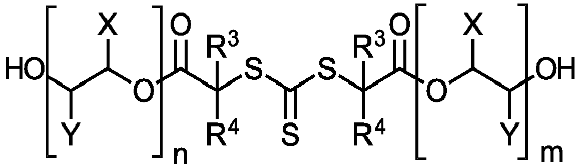 mit
- n und m: unabhängig voneinander 1 bis 100, bevorzugt 1 bis 50, besonders bevorzugt 1 bis 10 und ganz besonders bevorzugt 1 bis 4
- R³: ausgewählt wird aus der Gruppe bestehend aus Methyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
- R⁴: ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl Octyl, Phenyl, Benzyl und Cyclohexyl.
- X und Y: unabhängig voneinander ausgewählt wird aus der Gruppe bestehend aus Wasserstoff, Methyl, Ethyl, Propyl, Butyl, Phenyl, CH₂Cl, CH₂Br, CH₂OH, CH₂OCH₃, CH₂OCH₂CH₃, C(O)OCH₃, C(O)OCH₂CH₃, - (CH₂)₇CH₃, -(CH₂)₇C(O)OH, -(CH₂)₇C(O)OCH₃ und - (CH₂)₇C(O)OCH₂CH₃
und/oder Struktur (A-3) 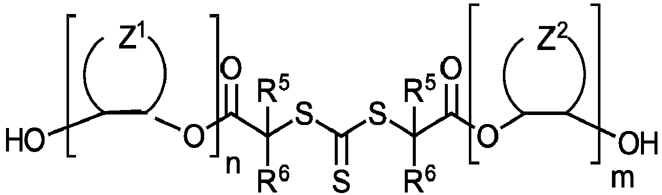 mit
- n und m: unabhängig voneinander 1 bis 100, bevorzugt 1 bis 50, besonders bevorzugt 1 bis 10 und ganz besonders bevorzugt 1 bis 4
- R⁵: ausgewählt wird aus der Gruppe bestehend aus Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
- R⁶: ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl,Octyl, Phenyl, Benzyl undCyclohexyl

Z¹, Z² unabhängig voneinander ausgewählt wird aus der Gruppe bestehend aus -(CH₂)₄-,-(CH₂)₅-, -(CH₂)₆-, -(CH₂)₇-, -(CH₂)₈-, -(CH₂(SO₂)CH₂)-, -(CH₂CH₂CHCHCH₂)- und-((CH₂)₃CHCH(CH₂)₃)-.

In einer bevorzugten Ausführungsform der Erfindung weist das H-funktionelles Trithiocarbonat (A) die Struktur (A-1) oder (A-2) mit:
R1 = R3 = Methyl,
R2 = R4 = Wasserstoff,
   und
X und Y unabhängig voneinander ausgewählt wird aus der Gruppe bestehend aus Wasserstoff und Methyl, Ethyl, Propyl, Butyl, Phenyl, -CH₂Cl, -CH₂Br, -CH₂OH, -CH₂OCH₃,-CH₂OCH₂CH₃, -C(O)OCH₃, -C(O)OCH₂CH₃, -(CH₂)₇CH₃, -(CH₂)₇COOH,-(CH₂)₇COOCH₃, -(CH₂)₇COOCH₂CH₃, und -CH₂OCH₂CHCH₂ auf.
In einer besonders bevorzugten Ausführungsform weist das Trithiocarbonat (A) die Struktur (A-2) auf und ist eine oder mehrere Verbindungen ausgewählt aus der Gruppe bestehend aus:
   (A-2.1(1)) 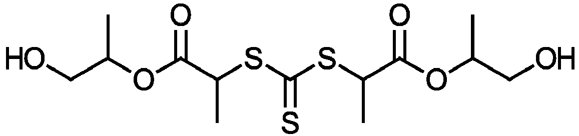
   , (A-2.1(2)) 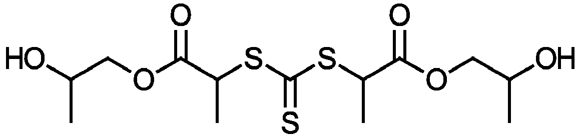
   , (A-2.1(3)) 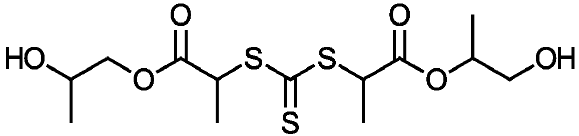 oder
   und (A-2.2)

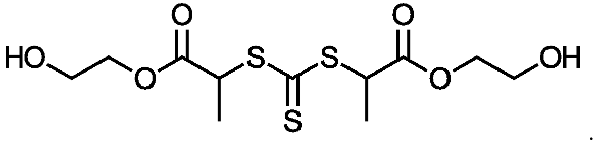

Die reversible Additions-Fragmentierungs-Kettenübertragungs-Polymerisation (engl. reversible addition-fragmentation chain transfer abgekürzt RAFT) ist gemäß des allgemeinen Fachwissens eine spezielle Form der kontrollierten freien radikalischen Polymerisation, wobei die Polymere mit wohldefinierter molarer Masse, definierten Polymerisationsgrad, geringer Polydispersität sowie bekannter Endfunktionalität zugänglich sind. Aufgrund der kontrollierten Reaktion sind auch Block-, Stern oder auch Kammpolymere definierter Struktur zugänglich, wobei die Reaktionskontrolle durch eine reversible Kettenübertragungsreaktion ermöglicht wird. Hierbei addiert sich eine wachsende Radikalkette an ein sogenanntes RAFT-Reagenz, wobei ein intermediäres Radikal resultiert. Basierend auf der Struktur der RAFT-Reagenzien kann dieses Radikal wiederum fragmentieren, wodurch wiederum ein RAFT-Reagenz und ein für das Polymerwachstum zur Verfügung stehendes Radikal zurückgebildet wird.
Mithilfe der Additions-Fragmentierungs-Kettenübertragungs-Polymerisation können Polymerisationsreaktionen mit zahlreichen Monomeren, meist Vinylgruppen-haltige Monomere unter milden Bedingungen ohne Einsatz von weiteren Reagenzien, beispielsweise retardierenden Reagenzien durchgeführt werden, wodurch die Synthesen die schnell und kostengünstig sind.

Eine weitere Ausführungsform der Erfindung betrifft das Verfahren zur Verfahren zur Herstellung eines H-funktionelles Trithiocarbonat (A) umfassend folgende Schritte:
i-1) Bereitstellung von einem oder mehreren Trithiocarbonat-Salz(en)
i-2) Umsetzung des Trithiocarbonat-Salzes aus Schritt i-1) mit einer aliphatischen, Halogen-haltigen H-funktionelle Verbindung zum H-funktionelle Trithiocarbonat (A) mit der Struktur (A-1).
i-3) Umsetzung der in Schritt i-2) erhaltenden Verbindung mit der Struktur (A-1) mit einem Alkylenoxid in Gegenwart eines Katalysators und eines Lösungsmittels zu einer Verbindung mit der Struktur (A-2),
wobei der Katalysator in Schritt i-3) eine Chrom(III)-haltige Verbindung ist und das in Schritt i-3) verwendete Lösungsmittel ein aprotisches Lösungsmittel ist.

### Schritt i-1)

In Schritt i-1 wird von einem oder mehreren Trithiocarbonat-Salz(en) bereitgestellt, wobei das Trithiocarbonat-Salz in Schritt i-1) durch basenkatalysierte Umsetzung von Schwefelkohlenstoff erhalten wird. Hierbei können verschiedene Basen verwendet werden, wobei bevorzugt Alkali- oder Erdalkalimetallhydroxide verwendet werden. In einer bevorzugten Ausführungsform ist die Base eine oder mehrere Verbindung(en) ausgewählt aus der Gruppe bestehend aus Lithiumhydroxid, Natriumhydroxid, Kaliumhydroxid, Magnesiumhydroxid, Calciumhydroxid, Strontiumhydroxid und Bariumhydroxid besonders bevorzugt Natriumhydroxid und Kaliumhydroxid.

### Schritt i-2)

In Schritt i-2) Trithiocarbonat-Salzes aus Schritt i-1) mit einer aliphatischen, Halogen-haltigen H-funktionelle Verbindung zum H-funktionelle Trithiocarbonat (A) mit der Struktur (A-1) (Intermediat) umgesetzt, wobei in der aliphatischen, Halogen-haltigen H-funktionellen Verbindung die Halogenhaltige Funktionalität mit der H-funktionellen Funktionalität über eine nicht-konjugierte kovalente Bindung verknüpft ist.
In einer bevorzugten Ausführungsform ist die Halogen-haltige H-funktionelle Verbindung eine oder mehrere Verbindung(en) ist und ausgewählt wird aus der Gruppe bestehend aus 2-Brompropionsäure, 2-Brombutansäure, 2-Brompentansäure, 2-Bromhexansäure, 3-Brombutansäure, 3-Brompentansäure, 3-Bromhexansäure, 2-Brom-2-methlypropionsäure, 2-Brom-2-methlybutansäure, 7-Bromölsäure, 8-Bromölsäure, 2-Brompropanol, 2-Brombutanol, 2-Brompentanol, 2-Bromhexanol, Bromcyclohexanol, 2-Bromheptanol, 2-Bromoctanol, 3-Brombutanol, 3-Brompentanol, 3-Bromhexanol, 3-Bromheptanol, 3-Bromoctanol, 2-Chlorpropionsäure, 2-Chlorbutansäure, 3-Chlorbutansäure, 2-Iodpropionsäure, 2-Iodbutansäure, 3-Iodbutansäure, 2-Chlorpropanol, 2-Chlorbutanol, 3-Chlorbutanol, 2-Iodpropanol, 2-Iodbutanol und 3-Iodbutanol.

Hierbei resultiert ein Trithiocarbonat - Intermediat, welches durch Substitutionsreaktion des Trithiocarbonat-Salzes aus Schritt-1) mit der aliphatischen, Halogen-haltigen H-funktionellen Verbindung gebildet wird. Als Nebenprodukt wird ein Halogenidsalz bevorzugt ein Alkalisalz- oder Erdalkalisalzhalogenid gebildet. In Abhängigkeit von der H-Funktion der verwendeten Halogen-haltige H-funktionelle Verbindung resultieren Trithiocarbonat - Intermediate mit Carbonsäuregruppen, Hydroxylgruppen und/oder Amingruppen.
Dieses in Schritt i-2) erhaltende Trithiocarbonat (A) mit der Struktur (A-1) (Intermediat) kann direkt als Reagenz für die reversible Additions-Fragmentierungs-Kettenübertragungs-Polymerisation verwendet werden oder in einem, an Schritt i-2), nachfolgenden Schritt i-3) durch Umsetzung dieses Intermediates mit Alkylenoxid in ein H-funktionelles Trithiocarbonat (A) mit der Struktur (A-2) umgestzt werden, wobei dieses H-funktionelles Trithiocarbonat (A) ebenfalls als Reagenz für die reversible Additions-Fragmentierungs-Kettenübertragungs-Polymerisation geeignet ist.

### Schritt i-3)

In Schritt i-3) wird die in Schritt i-2) erhaltende Verbindung mit der Struktur (A-1) mit einem Alkylenoxid in Gegenwart eines Katalysators und eines Lösungsmittels umgesetzt, wobei eine Verbindung mit der Struktur (A-2) (RAFT-Reagenz) resultiert.

Hierbei können für das erfindungsgemäße Verfahren verschiedene Alkylenoxide eingesetzt werden. Bevorzugt handelt es sich um eine oder mehrere Verbindungen ausgewählt aus der Gruppe bestehend aus Ethylenoxid, Propylenoxid, Epichlorhydrin, Epibromhydrin, Glycidol, Cyclohexenoxid, 9,10-Epoxystearinsäure, 9,10-Epoxyoctadecan-1-ol, Allylglycidylether, 2,3-Epoxybutan, und Styroloxid besonders bevorzugt Ethylenoxid und Propylenoxid.

Im erfindungsgemäßen Verfahren werden in Schritt i-1) als Alkylenoxid besonders bevorzugt Propylenoxid, Ethylenoxid oder ein Gemisch aus Propylenoxid und Ethylenoxid eingesetzt. Werden Gemische aus Ethylenoxid und Propylenoxid eingesetzt, weisen diese bevorzugt bis zu 50 Massen-% Ethylenoxid und besonders bevorzugt bis zu 30 Massen-% Ethylenoxid bezogen auf die Gesamtmasse des Gemisches aus Ethylenoxid und Propylenoxid auf. Ganz besonders bevorzugt wird ausschließlich Propylenoxid eingesetzt. Die Alkylenoxide können dem Reaktor als Einzelkomponenten oder als Gemisch zugeführt werden. Es ist ebenfalls möglich mehrere Alkylenoxide dem Reaktor nacheinander zuzuführen, womit sich Polyoxyalkylenketten mit Blockstruktur realisieren lassen. Bei der Dosierung mehrerer Alkylenoxide ist es möglich die Zusammensetzung des zugeführten Alkylenoxidstroms kontinuierlich oder instantan zu ändern.

In einer bevorzugten Ausführungsform ist die als Katalysator in Schritt i-3) verwendete Chrom(III)-haltige Verbindung eine oder mehrere Verbindung(en) ist ausgewählt wird aus der Gruppe bestehend aus Chromfluorid, Chromchlorid, Chrombromid, Chromiodid, Chromoxid, Chromacetylacetonat, Chromacetat, Chromformiat, Chromoxalat, Chromnitrat, Chromcarbonat, Chromsulfate und Chrompicolinat bevorzugt Chromchlorid.
In einer bevorzugten Ausführungsform des erfindungsgemäßen Verfahrens beträgt der Anteil von der als Katalysator in Schritt i-3) verwendete Chrom(III)-haltigen Verbindung 0,05-5 Gew.-% bezogen auf die eingesetzte Masse an Verbindung A-1, besonders bevorzugt 0,1-2 Gew.-%, ganz besonders bevorzugt 0,5-1,5 Gew.-%.
In einer Ausführungsform des erfindungsgemäßen Verfahrens wird Verbindung A-1, ein Lösungsmittel sowie ein Katalysator unter Inertgasbedingungen, bevorzugt Stickstoffgegenstrom vorgelegt. Anschließend wird das Alkylenoxid, bevorzugt Propylenoxid und/oder Ethylenoxid bei einer Temperatur (T₁) zudosiert und für eine Zeit (t₁) vermischt. Anschließend wird die Temperatur (T₂) erhöht und für eine weitere Zeit (t₂) gerührt. Nachdem das Reaktionsgemisch auf Raumtemperatur abkühlt wurde, wurde das Reaktionsgemisch mittels Dünnschichtchromatographie untersucht. In einer bevorzugten Ausführungsform des Verfahrens beträgt die Temperatur (T₂) 50 °C bis 200 °C, bevorzugt von 80 °C bis 150 °C, besonders bevorzugt von 90 °C bis 130 °C.
In einer bevorzugten Ausführungsform beträgt die Reaktionszeit (t₂) 2 h bis 20 h, bevorzugt 4 h bis 13 h.

### Lösungsmittel

In einer bevorzugten Ausführungsform wird Schritt i-3) als Lösungsmittel ein aprotisch-unpolares Lösungsmittels verwendet. In einer besonders bevorzugten Ausführungsform ist das aprotische-unpolare Lösungsmittel eine oder mehrere Verbindung(en) ist und ausgewählt wird aus der Gruppe bestehend aus Methylphenylether, Tetrahydrofuran, 2-Methyltetrahydrofuran, 2-Methoxy-2-methylpropan, Butanon, Aceton, Toluol, Benzol, Xylol, n-Pentan, n-Hexan, n-Heptan, Cyclopentan, Cyclohexan, Cycloheptan, Ethylacetat, iso-Propylacetat, n-Butylacetat Diethylether, Methylpropylether, sec-Butylmethylether, Dimethylacetal, Ethoxymethoxymethan, Dimethylether, Di-iso-propylether, 2,2-Dimethoxypropane, Chlorbenzol, Dichlorbenzol, Dichloromethan, Chloroform, 1,2-Dichlorethan, und 1,1,2,2-Tetrachlorethan, bevorzugt Methylphenylether, Ethylacetat, Toluol, Chlorbenzol, Dichlorbenzol und Xylol.

### Telechele Polyole (C)

Eine weitere Ausführungsform des erfindungsgemäßen Verfahrens umfasst ein Herstellungsverfahren eines telechelen Polyols, bevorzugt telechelen Diols, umfassend der Umsetzung eines H-funktionellen Trithiocarbonats (A) mit der Struktur (A-1) oder (A-2) oder (A-3) gemäß einem der Ansprüche 1 bis 3 oder ein H-funktionelles Trithiocarbonat (A) erhältlich nach einem der Ansprüche 4 bis 11 mit einem doppelbindungshaltigen Monomer (B) in Gegenwart eines Radikalstarters, wobei das doppelbindungshaltige Monomer (B) mit der folgenden Struktur aufweist: 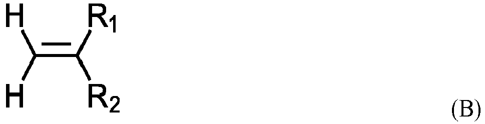 , wobei
R1= H, C1-C4 Alkylgruppen
R2= Halogen, Hydroxy-substituierte Arylgruppen, Hydroxy-unsubstituierte Arylgruppen, Halogen-substituierte C1-C10 Alkylgruppen, Hydroxy-substituierte C1-C10 Alkylgruppen, Alkylsäureestergruppen.
In einer Ausführungsform des erfindungsgemäßen Verfahrens ist der Radikalstarter eine oder mehrere Verbindung(en) und wird ausgewählt aus der Gruppe bestehend aus 2,2'-Azobis(2-methylpropionitril), 2,2'-Azobis(isobutyronitril), 2,2'-Azobis(2-methylbutyronitril), 2,2'-Azobis(2-cyanobutan), dimethyl 2,2'- Azobis(isobutyrat), 2,2'-Azobis(2,4-dimethylvaleronitril), 4,4'-Azobis(4-cyanovaleriansäure), 1,1'- Azobis(cyclohexanecarbonitril), 2-(t-Butylazo)-2-Cyanopropan, 2,2'-Azobis {2-Methyl-N-[1,1-bis(hydroxymethyl)-2-hydroxyethyl]propionamid}, 2,2'-Azobis[2-Methyl-N-(2-hydroxyethyl)propionamid], 2,2'-azobis(N,N'- dimethyleneiso-butyramidin)-dihydrochlorid, 2,2'-azobis(2-amidinopropan) dihydrochlorid, 2,2'-azobis(N,N'-dimethyleneisobutyramidin), , 2,2'-azobis(isobutyramid) dihydrat, 2,2'- azobis(2,2,4-trimethylpentan), 2,2'-azobis(2,4-dimethyl-4-methoxyvaleronitril), 2,2'-azobis(2-methylpropan), t-Butyl-peroxyacetat, t-Butylperoxybenzoat, t-Butyl-peroxyneodecanoat, t-Butylperoxyisobutyrat, t-Amyperoxypivalat, t-Butylperoxypivalat, Diisopropyl-peroxydicarbonat, Dicyclohexyl-peroxydicarbonat, Dicumyl-peroxid, Dibenzoyl-peroxid, Dilauroylperoxid, Kaliumperoxydisulfat, Ammoniumperoxydisulfat, Di-t-Butylhyponitrit und Dicumylhyponitrit bevorzugt 2,2'-Azobis(2-methylpropionitril), 2,2'-Azobis-2,4-dimethylvaleronitril, 2,2'-Azobisisobutyronitril und 2,2'-Azobis-2-methylbutyronitril.
In einer bevorzugten Ausführungsform des erfindungsgemäßen Verfahrens hat der Radikalstarter eine 10-Stunden-Halbwertszeit-Temperatur (engl. 10-hour half life temperature) im Reaktionsmedium von 25 °C bis 150 °C, bevorzugt 27 °C bis 130 °C, besonders bevorzugt 30 °C bis 110 °C. Dabei ist die 10-Stunden-Halbwertszeit-Temperatur als Temperatur definiert bei der nach 10 Stunden im Reaktionsmedium noch die Hälfte der eingesetzten Substanzmenge des Radikalstarters unzersetzt und reaktionsfähig vorliegt. Durch die Wahl des Radikalstarters wird eine Reaktionskontrolle der Umsetzung doppelbindungshaltiger Monomere (B) mit den H-funktionellen Trithiocarbonaten (A) A-1, A-2 und A-3 zu telechelen Polyolen, bevorzugt telechelen Diolen, erlangt. Der Begriff der Reaktionskontrolle umschließt Größen wie Reaktionsgeschwindigkeiten, Umsätze, Selektivitäten, Molekulargewichtsaufbau der jeweiligen Verbindungen, Molekulargewichtsverteilungen und Endgruppenfunktionalitäten aber ist nicht auf diese genannten Größen beschränkt..
In einer alternativen Ausführungsform kann auch ein Hydroxyl-Gruppen haltiger Radikalstarter verwendet werden, wie beispielsweise 2,2'-Azobis-2-methyl-N-(2-hydroxyethyl)propionamid.

### Doppelbindungshaltiges Monomer (B)

In einer bevorzugten Ausführungsform ist das Doppelbindungshaltige Monomer (B) eine oder mehrere Verbindung(en) ist und ausgewählt wird aus der Gruppe bestehend aus Vinylchlorid, Styrol, Butylacrylat, Acrylnitril, 2-Ethylhexylacrylat, Methylacrylat, Methylmethacrylat, Ethylacrylat, Ethylmethacrylat, Propylacrylat, Propylmethacrylat, Butylacrylat, Isobutylacrylat, Butylmethacrylat, Cyclohexylmethacrylat, (2-Ethylhexyl)acrylate, Isobornylmethacrylat, (Hydroxyethyl)methacrylat, N-Vinyl-pyrrolidon, N-Vinyl-pyrrolidon, N-Vinyl-caprolactam, N-Vinyl-caprolactam, N-Vinyl-imidazol, N-Vinyl-imidazol, N-Vinyl-N-Methylacetamid, Ethylvinylether, n-Butylvinylether, iso-Butylvinylether, Cyclohexylvinylether, 2-Ethylhexylvinylether, Dodecylvinylether, Octadecylvinylether, 1,4-Butanedioldivinylether, Diethyleneglycoldivinylether, Triethyleneglycoldivinylether , 1,4-Cyclohexanedimethanoldivinylether, Hydroxybutylvinylether, 1,4-Cyclohexanedimethanolmonovinylether, 1,2,4-Trivinylcyclohexane, 3-Aminopropylvinylether bevorzugt Styrol, Acrylnitril, Vinylacetat, Butylacrylat, Vinylchlorid, Methylacrylat, Ethylacrylat und Methylmethacrylat.bevorzugt Styrol, Acrylnitril, Vinylacetat, Butylacrylat, Vinylchlorid, Methylacrylat, Ethylacrylat und Methylmethacrylat, bevorzugt Styrol, Acrylnitril, Vinylacetat und Butylacrylat besonders bevorzugt Vinylchlorid, Styrol, Butylacrylat und Acrylnitril.

Ein weiterer Gegenstand ist auch eine telecheles Polyols (C), bevorzugt telecheles Diol herstellbar nach dem oben beschriebenen erfindungsgemäßen Verfahren, wobei in einer bevorzugten Ausführungsform das wobei das telechele Polyol (C) ein zahlenmittleres Molekulargewicht von 200 g/mol < Mn < 50000 g/mol bestimmt mit Gelpermeationschromatographie in Anlehnung an DIN 55672-1 und einem Polydispersitätsindex im Bereich von 1,1 bis 2,0, bevorzugt von 1,2 bis 1,8, wobei der Polydispersitätsindex mittels Gelpermeationschromatographie bestimmt wurde, aufweist..

In einer weiteren Ausführungsform können die erfindungsgemäßen telechelen Polyole mit OHreaktiven Verbindungen, welche OH-Gruppen-reaktive funktionelle Gruppen enthalten und in Polyadditionsreaktionen oder Polykondensationsreaktion umgesetzt werden.

In einer Ausführungsform des erfindungsgemäßen Verfahrens wird das erfindungsgemäße telechelen Polyole mit mindenstens einer Dicarboncarbonsäure-Komponente, mindestens einer Carbonsäureanhydrid-Komponente, Phosgen und/oder mindestens einer Isocyanatgruppenhaltigen Verbindungen ungesetzt.

Weiterhin ist Kohlensäure im Rahmen dieser Erfindung ebenfalls eine Dicarbonsäure, die bevorzugt als Dicarbonsäureäquivalent in Form von Dialkylestern, beispielsweise als Dimethylcarbonat, oder als Diarylester, beispielsweise als Diphenylcarbonat eingesetzt werden kann.
Bevorzugt bestehen Polyesterpolyole aus alternierenden Polycarbonsäure-Komponenten und Polyolkomponenten. Als Polycarbonsäure-Komponenten können z.B. Oxalsäure, Malonsäure, Bernsteinsäure, Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Korksäure, Nonandicarbonsäure, Decandicarbonsäure, Dodecandicarbonsäure, Tetradecandicarbonsäure, Hexadecandicarbonsäure, 2-Methylbernsteinsäure, 3,3-Diethylglutarsäure, sowie Dimerfettsäuren und/oder 2,2-Dimethylbernsteinsäure. Als Säurequelle können auch die entsprechenden analogen Anhydride und/oder niedermolekulare Ester verwendet werden.

In einer bevorzugten Ausführungsform des erfindungsgemäßen Verfahrens ist die gegenüber OH-Gruppen reaktive Verbindung mindestens eine Isocyanatgruppen-haltige Verbindung. Geeignete Polyisocyanate der Isocyanatgruppen-haltige Verbindung sind die dem Fachmann an sich bekannten aromatischen, araliphatischen, aliphatischen oder cycloali-pha-tischen Polyisocyanate, welche auch Iminooxadiazindion , Isocyanurat , Uret¬dion-, Urethan , Allophanat , Biuret , Harnstoff-, Oxadiazintrion, Oxazolidinon-, Acylharnstoff-, Carbamat- und/oder Carbodiimid-Strukturen aufweisen können. Diese können einzeln oder in beliebigen Mischungen untereinander eingesetzt werden.
Die vorstehend genannten Polyisocyanate basieren dabei auf dem Fachmann an sich bekannten Diisocyanaten oder Triisocyanaten oder höher funktionellen Isocyanaten mit aliphatisch, cycloaliphatisch, araliphatisch und/oder aromatisch gebundenen Isocyanatgruppen, wobei es unerheblich ist, ob diese unter Verwendung von Phosgen oder nach phosgenfreien Verfahren hergestellt wurden. Beispiele für solche Diisocyanate oder Triisocyanate oder höher funktionellen Isocyanaten sind 1,4-Diisocyanatobutan, 1,5-Diisocyanatopentan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan (Desmodur® W, Covestro AG, Leverkusen, DE), 4-Isocyanatomethyl-1,8-octandiisocyanat (Triisocyanatononan, TIN), ω,ω'-Diisocyanato-1,3-dimethylcyclohexan (H₆XDI), 1-Isocyanato-1-methyl-3-isocyanato-methylcyclohexan, 1-Isocyanato-1-methyl-4-isocyanato-methylcyclohexan, Bis-(isocyanatomethyl)-norbornan, 1,5-Naphthalen-diisocyanat, 1,3- und 1,4-Bis-(2-isocyanato-prop-2-yl)-benzol (TMXDI), 2,4- und 2,6-Diisocyanatotoluol (TDI) insbesondere das 2,4 und das 2,6-Isomere und technische Gemische der beiden Isomeren, 2,4'- und 4,4'-Diisocyanatodiphenylmethan (MDI), 1,5-Diisocyanatonaphthalin, 1,3-Bis(isocyanato-methyl)benzol (XDI) sowie beliebige Mischungen genannter Verbindungen, sowie durch Dimerisierung oder Trimerisierung oder höhere Oligomerisierung der genannten Isocyanate erhaltene polyfunktionelle Isocyanate, enthaltend Isocyanurat-Ringen, Iminooxadiazindion-Ringe, Uretdion-Ringe, Urethoniminringe, sowie durch Addukt-Bildung der genannten Isocyanate an Mischungen verschiedener mehr als difunktioneller Alkohole, wie 1,1,1-Trimethylolpropan (TMP), Trimethylolethan (TME) oder Pentaerythrit, gewonnene polyfunktionelle Isocyanate.
Bevorzugt weisen die Verbindungen der Isocyanatgruppen-haltige Verbindung dabei einen Gehalt an Iso¬cyanatgruppen von 2 Gew.-% bis 60 Gew.-%, bevorzugt von 15 Gew.-% bis 50 Gew.-%.
Besonders bevorzugt werden in Isocyanatgruppen-haltige Verbindung Polyisocyanate oder Polyiso¬cyanatgemische der vorstehend genannten Art mit ausschließlich aliphatisch und/oder aromatisch ge¬bundenen Iso¬cyanatgruppen eingesetzt.
Ganz besonders bevorzugt enthält die Isocyanatgruppen-haltige Verbindung ausgewählt aus der Gruppe bestehend aus HDI, MDI, TDI, NDI deren Trimerisierungs- oder höheren Oligomerisierungsprodukte und deren Addukte.

Die telechelen Polyole können allein oder gegebenenfalls im Gemisch mit weiteren isocyanatreaktiven Komponenten mit organischen Polyisocyanaten, gegebenenfalls in Gegenwart von Treibmitteln, in Gegenwart von Katalysatoren und gegebenenfalls mit weiteren Zusatzstoffen wie z.B. Zellstabilisatoren zur Reaktion gebracht werden und so als Komponenten von massiven oder geschäumten Polyurethanen, insbesondere Polyurethan-Weichschaum wie beispielsweise Polyurethan-Weichblockschaum und Polyurethan-Weichformschaum, dienen. Polyurethane, bevorzugt massive oder geschäumte Polyurethane, insbesondere Polyurethan-Weichschäume wie beispielsweise Polyurethan-Weichblockschäume und Polyurethan-Weichformschäume, enthaltend die erfindungsgemäßen telechelen Polyole sind ebenfalls Gegenstand der Erfindung.
Eine weitere Ausführung der Erfindung betrifft ein Verfahren zur Herstellung eines Trithiocarbonat-freien, telechelen Polyols (D), bevorzugt telechelen Diols, durch Umsetzung des telechelen Polyols (C), bevorzugt telechelen Diols mit einem Oxidationsmittel und optional eines Radikalstarters und optional eines Lösungsmittels.
In einer bevorzugten Ausführung ist das Oxidationsmittel eine oder mehrer(e) Verbindung(en) und wird ausgewählt aus der Gruppe bestehend aus Ozon, Sauerstoff, Wasserstoffperoxid, aliphatisch und aromatische Persäuren, wie beispielsweise Peressigsäure, Trifluoroperessigsäure, Peroxybenzimidsäure (Payne Reagenz), Magnesium monoperpthalate, 2,4-Dinitroperbenzoesäure und meta-Chlorperbenzoesäure (MCPBA), Iod, Periodsäure, Chlor, Perchlorsäure, Brom, Perbromsäure, Natriumhypochlorite, Kaliumhypochlorite, Perborate, Percarbonate, Permanganate, Kaliumnitrat, Distickstoffoxid, Salpetersäure, Peroxomonoschwefelsäure, Kaliumperoxomonosulfat und Kaliumnitrat.
In einer bevorzugten Ausführungsform ist das Lösungsmittel ein organisches Lösungsmittel oder Wasser, besonders bevorzugt cyclische und lineare Ether, ganz besonders bevorzugt Tetrahydrofuran
In einer bevorzugten Ausführungsform ist der Radikalstarter für die Herstellung des Trithiocarbonat-freien, telechelen Polyols (D), bevorzugt telechelen Diols, identisch mit dem für das telechele Polyol, bevorzugt telechele Diol.

In einer ersten Ausführung betrifft die Erfindung ein H-funktionelles Trithiocarbonat (A) als Reagenz für die reversible Additions-Fragmentierungs-Kettenübertragungs-Polymerisation (RAFT), wobei das H-funktionelle Trithiocarbonat (A) die Struktur (A-1) 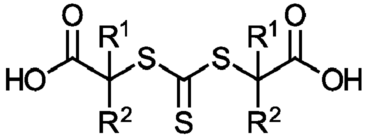 mit
- R¹: ausgewählt wird aus der Gruppe bestehend aus Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
- R²: ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl Octyl, Phenyl, Benzyl und Cyclohexyl
und/oder Struktur (A-2) 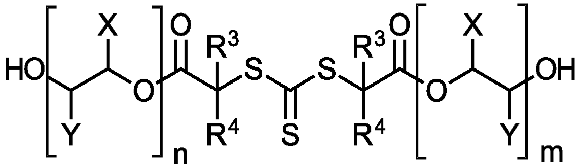 mit
- n und m: unabhängig voneinander 1 bis 100, bevorzugt 1 bis 50, besonders bevorzugt 1 bis 10 und ganz besonders bevorzugt 1 bis 4
- R³: ausgewählt wird aus der Gruppe bestehend aus Methyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
- R⁴: ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl Octyl, Phenyl, Benzyl und Cyclohexyl.
- X und Y: unabhängig voneinander ausgewählt wird aus der Gruppe bestehend aus Wasserstoff, Methyl, Ethyl, Propyl, Butyl, Phenyl, CH₂Cl, CH₂Br, CH₂OH, CH₂OCH₃, CH₂OCH₂CH₃, C(O)OCH₃, C(O)OCH₂CH₃, -(CH₂)₇CH₃, - (CH₂)₇C(O)OH, -(CH₂)₇C(O)OCH₃ und -(CH₂)₇C(O)OCH₂CH₃
und/oder Struktur (A-3) 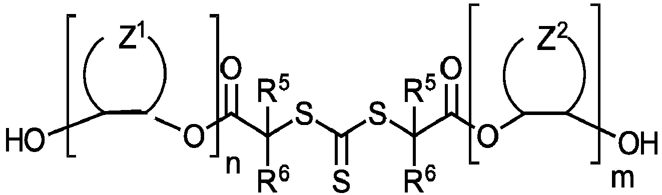 mit
n und m unabhängig voneinander 1 bis 100, bevorzugt 1 bis 50, besonders bevorzugt 1 bis 10 und ganz besonders bevorzugt 1 bis 4
- R⁵: ausgewählt wird aus der Gruppe bestehend aus Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
- R⁶: ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl,Octyl, Phenyl, Benzyl undCyclohexyl
- Z¹, Z²: unabhängig voneinander ausgewählt wird aus der Gruppe bestehend aus - (CH₂)₄-, -(CH₂)₅-, -(CH₂)₆-, -(CH₂)₇-, -(CH₂)₈-, -(CH₂(SO₂)CH₂)-, - (CH₂CH₂CHCHCH₂)- und -((CH₂)₃CHCH(CH₂)₃)- aufweist.

In einer zweiten Ausführung betrifft die Erfindung ein H-funktionelles Trithiocarbonat (A) gemäß der ersten Ausführung, wobei das Trithiocarbonat (A) folgende die Struktur (A-1) oder (A-2) mit
R¹ = R³ = Methyl,
R² = R⁴ = Wasserstoff,
und
- X und Y: unabhängig voneinander ausgewählt wird aus der Gruppe bestehend aus Wasserstoff und Methyl, Ethyl, Propyl, Butyl, Phenyl, -CH₂Cl, -CH₂Br, -CH₂OH, - CH₂OCH₃, -CH₂OCH₂CH₃, -C(O)OCH₃, -C(O)OCH₂CH₃, -(CH₂)₇CH₃, - (CH₂)₇COOH, -(CH₂)₇COOCH₃, -(CH₂)₇COOCH₂CH₃, und -CH₂OCH₂CHCH₂.
aufweist.

In einer dritten Ausführung betrifft die Erfindung ein H-funktionelles Trithiocarbonat (A) gemäß der ersten oder zweiten Ausführung, wobei das Trithiocarbonat (A) die Struktur (A-2) aufweist und eine oder mehrere Verbindungen ausgewählt aus der Gruppe bestehend aus:
(A-2.1(1)) 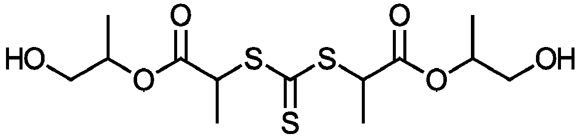
, (A-2.1(2)) 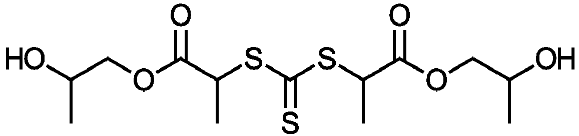
, (A-2.1(3)) 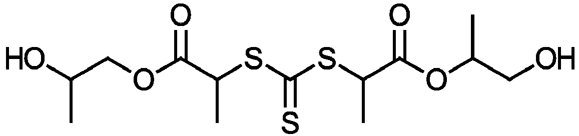 oder
und (A-2.2)

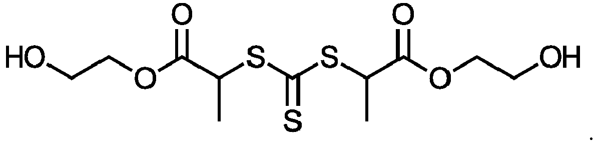

In einer vierten Ausführung betrifft die Erfindung ein Verfahren zur Herstellung eines H-funktionelles Trithiocarbonat (A) umfassend folgende Schritte:
i-1) Bereitstellung von einem oder mehreren Trithiocarbonat-Salz(en)
i-2) Umsetzung des Trithiocarbonat-Salzes aus Schritt i-1) mit einer aliphatischen, Halogen-haltigen H-funktionelle Verbindung zum H-funktionelle Trithiocarbonat (A) mit der Struktur (A-1).
i-3) Umsetzung der in Schritt i-2) erhaltenden Verbindung mit der Struktur (A-1) mit einem Alkylenoxid in Gegenwart eines Katalysators und eines Lösungsmittels zu einer Verbindung mit der Struktur (A-2),
wobei der Katalysator in Schritt i-3) eine Chrom(III)-haltige Verbindung ist und das in Schritt i-3) verwendete Lösungsmittel ein aprotisches Lösungsmittel ist.

In einer fünften Ausführung betrifft die Erfindung ein Verfahren gemäß der vierten Ausführung, wobei das Trithiocarbonat-Salz in Schritt i-1) durch basenkatalysierte Umsetzung von Schwefelkohlenstoff erhalten wird.

In einer sechsten Ausführung betrifft die Erfindung ein Verfahren gemäß der vierten oder fünften Ausführung, wobei die Halogen-haltige H-funktionelle Verbindung eine oder mehrere Verbindung(en) ist und ausgewählt wird aus der Gruppe bestehend aus 2-Brompropionsäure, 2-Brombutansäure, 2-Brompentansäure, 2-Bromhexansäure, 3-Brombutansäure, 3-Brompentansäure, 3-Bromhexansäure, 2-Brom-2-methlypropionsäure, 2-Brom-2-methlybutansäure, 7-Bromölsäure, 8-Bromölsäure, 2-Brompropanol, 2-Brombutanol, 2-Brompentanol, 2-Bromhexanol, Bromcyclohexanol, 2-Bromheptanol, 2-Bromoctanol, 3-Brombutanol, 3-Brompentanol, 3-Bromhexanol, 3-Bromheptanol, 3-Bromoctanol, 2-Chlorpropionsäure, 2-Chlorbutansäure, 3-Chlorbutansäure, 2-Iodpropionsäure, 2-Iodbutansäure, 3-Iodbutansäure, 2-Chlorpropanol, 2-Chlorbutanol, 3-Chlorbutanol, 2-Iodpropanol, 2-Iodbutanol und 3-Iodbutanol.

In einer siebten Ausführung betrifft die Erfindung ein Verfahren gemäß einer der vierten bis sechsten Ausführung, wobei das Alkylenoxid in Schritt i-3) eine oder mehrere Verbindung(en) ist und ausgewählt wird aus der Gruppe bestehend aus Ethylenoxid, Propylenoxid, Epichlorhydrin, Epibromhydrin, Glycidol, Cyclohexenoxid, 9,10-Epoxystearinsäure, 9,10-Epoxyoctadecan-1-ol, Allylglycidylether, 2,3- Epoxybutan, und Styroloxid bevorzugt Ethylenoxid und Propylenoxid.

In einer achten Ausführung betrifft die Erfindung ein Verfahren gemäß einer der vierten bis siebten Ausführung, wobei die Reaktionstemperatur T₂ in Schritt i-3) von 50 °C bis 200 °C, bevorzugt von 80 °C bis 150 °C, besonders bevorzugt von 90 °C bis 130 °C beträgt.

In einer neunten Ausführung betrifft die Erfindung ein Verfahren gemäß der Ausführung, wobei die als Katalysator in Schritt i-3) verwendete Chrom(III)-haltige Verbindung eine oder mehrere Verbindung(en) ist ausgewählt wird aus der Gruppe bestehend aus Chromfluorid, Chromchlorid, Chrombromid, Chromiodid, Chromoxid, Chromacetylacetonat, Chromacetat, Chromformiat, Chromoxalat, Chromnitrat, Chromcarbonat, Chromsulfate und Chrompicolinat bevorzugt Chromchlorid.

In einer zehnten Ausführung betrifft die Erfindung ein Verfahren gemäß einer der vierten bis neunten Ausführung, wobei das in Schritt i-3) verwendete Lösungsmittel ein aprotisch-unpolares Lösungsmittel ist.

In einer elften Ausführung betrifft die Erfindung ein Verfahren gemäß der zehnten Ausführung, wobei das aprotisch-unpolare Lösungsmittel Lösungsmittel eine oder mehrere Verbindung(en) ist und ausgewählt wird aus der Gruppe bestehend aus Methylphenylether, Tetrahydrofuran, 2-Methyltetrahydrofuran, 2-Methoxy-2-methylpropan, Butanon, Aceton, Toluol, Benzol, Xylol, n-Pentan, n-Hexan, n-Heptan, Cyclopentan, Cyclohexan, Cycloheptan, Ethylacetat, *iso-*Propylacetat, *n*-Butylacetat Diethylether, Methylpropylether, sec-Butylmethylether, Dimethylacetal, Ethoxymethoxymethan, Dimethylether, Di-iso-propylether, 2,2-Dimethoxypropane, Chlorbenzol, Dichlorbenzol, Dichloromethan, Chloroform, 1,2-Dichlorethan, und 1,1,2,2-Tetrachlorethan, bevorzugt Methylphenylether, Ethylacetat, Toluol, Chlorbenzol, Dichlorbenzol und Xylol.

In einer zwölften Ausführung betrifft die Erfindung ein Verfahren zur Herstellung eines telechelen Polyols (C), bevorzugt telechelen Diols, umfassend der Umsetzung eines H-funktionellen Trithiocarbonats (A) mit der Struktur (A-1) oder (A-2) oder (A-3) gemäß einem der Ansprüche 1 bis 3 oder ein H-funktionelles Trithiocarbonat (A) erhältlich nach einem der Ansprüche 4 bis 11 mit einem doppelbindungshaltigen Monomer (B) in Gegenwart eines Radikalstarters, wobei das doppelbindungshaltige Monomer (B) mit der folgenden Struktur aufweist: 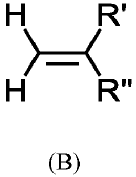 wobei
R'= H, C1-C4 Alkylgruppen
R"= Halogen, Hydroxy-substituierte Arylgruppen, Hydroxy-unsubstituierte Arylgruppen, Halogen-substituierte C1-C10 Alkylgruppen, Hydroxy-substituierte C1-C10 Alkylgruppen, Alkylsäureestergruppen.
In einer dreizehnten Ausführung betrifft die Erfindung ein Verfahren gemäß der zwölften Ausführung, wobei das doppelbindungshaltigen Monomer (B) eine oder mehrere Verbindung(en) ist und ausgewählt wird aus der Gruppe bestehend aus Vinylchlorid, Styrol, Butylacrylat, Acrylnitril, 2-Ethylhexylacrylat, Methylacrylat, Methylmethacrylat, Ethylacrylat, Ethylmethacrylat, Propylacrylat, Propylmethacrylat, Butylacrylat, Isobutylacrylat, Butylmethacrylat, Cyclohexylmethacrylat, (2-Ethylhexyl)acrylate, Isobornylmethacrylat, (Hydroxyethyl)methacrylat, N-Vinyl-pyrrolidon, N-Vinyl-pyrrolidon, N-Vinyl-caprolactam, N-Vinyl-caprolactam, N-Vinyl-imidazol, N-Vinyl-imidazol, N-Vinyl-N-Methylacetamid, Ethylvinylether, n-Butylvinylether, *iso*-Butylvinylether, Cyclohexylvinylether, 2-Ethylhexylvinylether, Dodecylvinylether, Octadecylvinylether, 1,4-Butanedioldivinylether, Diethyleneglycoldivinylether, Triethyleneglycoldivinylether , 1,4-Cyclohexanedimethanoldivinylether, Hydroxybutylvinylether, 1,4-Cyclohexanedimethanolmonovinylether, 1,2,4-Trivinylcyclohexane, 3-Aminopropylvinylether bevorzugt Styrol, Acrylnitril, Vinylacetat, Butylacrylat, Vinylchlorid, Methylacrylat, Ethylacrylat und Methylmethacrylat.

In einer vierzehnten Ausführung betrifft die Erfindung ein telecheles Polyol (C), bevorzugt telecheles Diol herstellbar nach einem Verfahren gemäß der zwölften oder dreizehnten Ausführung.

In einer fünfzehnten Ausführung betrifft die Erfindung ein telecheles Polyol (C), bevorzugt telecheles Diol gemäß der vierzehnten Ausführung, wobei das telechele Polyol ein zahlenmittleres Molekulargewicht von 200 g/mol < Mₙ < 50000 g/mol bestimmt mit Gelpermeationschromatographie in Anlehnung an DIN 55672-1 und einem Polydispersitätsindex im Bereich von 1,1 bis 2,0, bevorzugt von 1,2 bis 1,8, wobei der Polydispersitätsindex mittels Gelpermeationschromatographie bestimmt wurde, aufweist.

In einer sechzehnten Ausführung betrifft die Erfindung ein Verfahren zur Herstellung eines Trithiocarbonat-freien Telechelen Polyols (D), bevorzugt telechelen Diols, durch Umsetzung des telechelen Polyols (C), bevorzugt telecheles Diol gemäß der vierzehnten oder fünfzehnten mit einem Oxidationsmittel.

### Beispiele

### Methoden:

### Dünnschichtchromatographie (kurz DC)

DC wurde zur Reaktionskontrolle in Schritt i-3) (Darstellung von Verbindung A-2) genutzt. Es wurden 40 x 80 mm-DC-Plättchen des Typs Polygram SIL G/UV₂₅₄ mit 0,2 mmm Kieselgel 60 mit Fluoreszenz-Indikator der Firma Macherey-Nagel, eine UV-Lampe (λ = 254 nm) sowie eine Standard-DC-Kammer verwendet. Die Reaktionsgemische wurden ohne Aufreinigung direkt auf die DC-Plättchen geträufelt und sofern nicht anders beschrieben wurde ein 1:1-Gemisch (Vol./Vol.) aus Cyclohexan und Ethylacetat als Laufmittel verwendet.

¹H-NMR-Spektroskopie (Protonenresonanzspektroskopie, kurz NMR): Die Messungen erfolgten auf einem Bruker AV400 (400 MHz); alle chemischenVersichbungen sind gegen Tetramethylsilan (TMS) referenziert und werden in ppm (σ) angegeben. Die Multiplizitäten der ¹H-NMR-Spektren wurden wie folgt abgekürzt: Singulett, s; Dublett, *d*; Triplett, *t*; Dublett vom Dublett, *dd,* Quartett, *q* und Multiplett, *m.*

### Gelpermeationschromatographie (GPC)

Die Aufnahme der GPC-Chromatogramme erfolgte in Anlehnung an DIN 55672-1 auf einem Agilent 1200 Series, Detektion über RID; Elutionsmittel: Tetrahydrofuran, Flussrate 1.0 mL/min; Säulenkombination: PSS SDV Vorsäule 8x50 mm (5 µm), 2× PSS SDV linear S 8×300 ml (5 µm). Polystyrol bekannter 5 Molmasse der Firma "PSS Polymer Standards Service" wurden zur Kalibrierung verwendet. Es werden zahlenmittlere Molgewichte (Mₙ) sowie massenmittlere Molgewichte (M_{w}) sowie die Polydispersität (PDI) als Quotient aus M_{w} und Mₙ ermittelt.

**Eingesetzte Rohstoffe**

| | | |
|---|---|---|
| entionisiertes Wasser | | (aus Laborringleitung) |
| Kaliumhydroxid (KOH) | Reinheit 98 % | *Sigma-Aldrich* Chemie GmbH |
| Kohlenstoffdisulfid (CS₂) | Reinheit 99 % | *Sigma-Aldrich* Chemie GmbH |
| 2-Brompropionsäure | Reinheit > 99 % | Sigma Aldrich Chemie GmbH |
| Dichlormethan (CH₂Cl₂) | Reinheit ≥ 99,5 % | Sigma Aldrich Chemie GmbH |
| konzentrierte Salzsäure (HCl) | 37-%ige aq. Lösung | Carl Roth GmbH + Co. KG |
| Stickstoff 5.0 | Reinheit ≥ 99,999 % | Linde AG |
| Chrom(III)-chlorid (Cr(III)Cl₃) | Reinheit 99 % | Acros Organics BVBA |
| Triethylendiamin (DABCO) | Reinheit 99 % | abcr GmbH |
| Magnesium(II)-hydroxid (Mg(OH)₂) | Reinheit 95 | % *Sigma-Aldrich* Chemie GmbH |
| Cyclohexan (C₆H₁₂) | Reinheit 99.5 % | *Sigma-Aldrich* Chemie GmbH |
| Ethylacetat (EtOAc) | Reinheit 99.8 % | *Sigma-Aldrich* Chemie GmbH |
| Silica-Gel 60 (SiO₂) | hochrein | *Sigma-Aldrich* Chemie GmbH |
| Toluol | Reinheit 99.8 % | *Sigma-Aldrich* Chemie GmbH |
| Tetrahydrofuran (THF) | Reinheit 99.9 % | *Sigma-Aldrich* Chemie GmbH |
| Dimethylacetamid (DMAc) | Reinheit 99 % | *Sigma-Aldrich* Chemie GmbH |
| Propylenoxid (PO) | Reinheit 99.5 % | *Sigma-Aldrich* Chemie GmbH |
| n-Hexan (C₆H₁₄) | Reinheit 95 % | *Sigma-Aldrich* Chemie GmbH |
| trockenes, deuteriertes | | |
| Dimethylsulfoxid (DMSO-d₆) | 99,9 Atom-% D | *Sigma-Aldrich* Chemie GmbH |
| Iod | Reinheit ≥ 99,8 % | *Sigma-Aldrich* Chemie GmbH |
| Triphenylphosphin | Reinheit 99 % | *Sigma-Aldrich* Chemie GmbH |
| Imidazol | Reinheit ≥ 99 % | *Sigma-Aldrich* Chemie GmbH |
| trockenes Ethylenglycol | Reinheit 99.8 % | *Sigma-Aldrich* Chemie GmbH |
| 1,4-Dioxan (Dioxan) | Reinheit 99,8 % | *Sigma-Aldrich* Chemie GmbH |
| 2,2'-Azobis(2,4-dimethylvaleronitril) | (V-65) | Wako Chemicals GmbH |
| 2,2'-Azobis(2-methyl- N-(2-hydroxyethyl) propionamid) | (VA-086) | Wako Chemicals GmbH |
| Styrol | Reinheit ≥ 99 % | *Sigma-Aldrich* Chemie GmbH |
| Acrylnitril | Reinheit ≥ 99 % | *Sigma-Aldrich* Chemie GmbH |
| Dimethylsulfoxid (DMSO) | Reinheit ≥ 99 % | *Sigma-Aldrich* Chemie GmbH |
| Methanol | Reinheit 99 % | abcr GmbH |
| Acrylsäurebutylester (Butylacrylat) | Reinheit ≥ 99 % | *Sigma-Aldrich* Chemie GmbH |
| *Chlorbenzol* | Reinheit 99 % | abcr GmbH |

### Herstellung H-funktionelles Trithiocarbonat (A):

### Schritt i-1) Bereitstellung des Trithiocarbonat-Salzes am Beispiel von K₂CS₃

Die Darstellung von Kaliumtrithiocarbonat erfolgte analog der Offenbarung in Macromolecules, 2012, 45, 4958-4965. In einen Kolben wurden 1230 mL entionisiertes Wassser sowie 215 g KOH unter Rührung vorgelegt und dann 273 g Kohlenstoffdisulfid unter Rühren hinzufügt.

### Schritt i-2) Umsetzung des Trithiocarbonat-Salzes mit 2-Brompropionsäure zum H-funktionellen Trithiocarbonat-Intermediat (Verbindung A-1.1)

Die Darstellung der Verbindung A-1.1 erfolgte analog der Offenbarung in Macromolecules, 2012, 45, 4958-4965. Bei Raumtemperatur wurden 250 g 2-Brompropionsäure tropfenweise zu dem in Schritt i-1) erhaltenen Reaktionsgemisch hinzugegeben und anschließend für 72 h gerührt. Das resultierende Reaktionsgemisch wurde zweifach mit Dichlormethan gewaschen und mit konzentriertem HCl angesäuert, filtriert und getrocknet. Flüchtige Bestandteile wurden am Rotationsverdampfer abgetrennt. Die Ausbeute der Verbindung A-1.1 betrug 45.32 g.

### Schritt i-3) Umsetzung der in Schritt i-2) erhaltenden Verbindung A-1.1 mit einem Propylenoxid in Gegenwart eines Katalysators und eines Lösungsmittels zu Verbindung A-2.1 (RAFT-Reagenz 1)

In einen 300 mL Druckreaktor wurden die Verbindung A-1, ein Lösungsmittel sowie ein Katalysator unter Stickstoffgegenstrom vorgelegt. Anschließend wurden unter Rühren (500 min⁻¹) Propylenoxid mittels einer HPLC-Pumpe zu dosiert (1-2 g/min), für 3,5 h (t1) bei 80 °C (T1) gerührt und anschließend die Temperatur (T₂) erhöht und für eine weitere Zeit (t₂) gerührt (Details siehe Tab. 1). Nachdem das Reaktionsgemisch auf Raumtemperatur abkühlen wurde, wurde das Reaktionsgemisch mittels Dünnschichtchromatographie untersucht. Zwecks Isolation des Zielprodukts wurde das erhaltene Reaktionsgemisch über eine Kurzwegsäule aus Silica-Gel 60 filtriert. Die Säule wurde anschließend mit wenig Hexan und dann Ethylacetat gespült. Die vereinigten organischen Phasen wurden am Rotationsverdampfer von Lösungsmitteln und verdampfbaren Komponenten befreit. Die so gewonnene Verbindung A-2.1 (Fig. 2) wurde mittels ¹H-NMR charakterisiert und die Ausbeute bestimmt.
¹H-NMR (400 MHz, DMSO-d₆): 4.9-4.6 (m, 1.70), 4.0-3.8 (m, 2.35), 3.4 (m, 1.0), 1.55 (t, 3.23), 1.13 (d, 1.20), 1.05 (dd, 2.19) ppm.

**Tabelle 1: Vergleichs- und Ausführungsbeispiele zu Schritt i-3) Umsetzung der Verbindung A-1 mit Propylenoxid (PO) in Gegenwart von Katalysator und Lösungsmittel zu Verbindung A-2.1 mit t1 = 3,5 h und T1 = 80 °C. ^{a)} Mittels ¹H-NMR wurde ein Gehalt an 35 % primären und 65 % sekundären OH-Gruppen bestimmt. Zur Methode und NMR-Referenzverschiebungen siehe J. Mol. Catal. B Enzym., 2010, 62, 80-89.**

| Beispiel | A-1 [g] | Lösungsmittel ([g]) | Katalysator ([g]) | PO [g] | t₂ [h] | T₂ [°C] | Ausbeute A-2.1 [%] |
|---|---|---|---|---|---|---|---|
| 1 (Vgl.) | 2.5 | Toluol (100) | Mg(OH)₂ (0,24) | 5.8 | 5 | 110 | 2 |
| 2 (Vgl.) | 2.5 | THF (100) | Mg(OH)₂ (0,24) | 5.8 | 5 | 110 | 0 |
| 3 (Vgl.) | 2.5 | Toluol (100) | DABCO (0,24) | 5.8 | 15 | 100 | 2 |
| 4 (Vgl.) | 2.5 | DMAc (100) | DABCO (0,24) | 5.8 | 15 | 100 | 0 |
| 5 | 1.25 | Toluol (50) | CrCl₃ (0,016) | 5.8 | 5 | 100 | 80^{a)} |
| 6 | 18.75 | Toluol (120) | CrCl₃ (0,16) | 58 | 6 | 100 | 86 |
| 7 | 18.75 | Toluol (100) | CrCl₃ (0,12) | 58 | 11 | 90 | 86 |

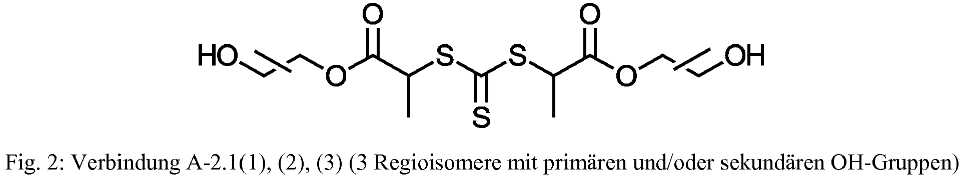

### Beispiel 8 zur Darstellung von Verbindung (A-2.2)

Die Darstellung von Verbindung A-2.2 aus Verbindung A-1 und Diol erfolgte in Analogie *zu* J. Org. Chem., 2011, 76, 2277-2281. Zu einem Gemisch aus 200 mL Dichlormethan und 11,52 g Iod wurden 10,16 g Triphenylphosphin und 8,98 g Imidazol bei Raumtemperatur unter Rühren zugegeben. Anschließend wurden 5,08 g Verbindung A-1 addiert und 5 min gerührt. Nach der Zugabe von 36,8 g Ethylenglycol wurde für 24 h bei Raumtemperatur gerührt. Das so gewonnene Reaktionsgemisch wurde mit Wasser extrahiert, die Dichlormethan-Phase isoliert und am Rotationsverdampfer von flüchtigen Bestandteilen befreit. Aus dem Rückstand wurde mittels präparativer Säulenchromatographie (stationäre Phase: Silica-Gel 60, Laufmittel: 3:1-Gemisch (Vol./Vol.) aus n-Hexan und Ethylacetat) das Zielprodukt Verbindung A-2 mit 48 % isoliert.
¹H-NMR (400 MHz, DMSO-d₆): 4.75 (t, 1H), 4.70 (m, 1H), 4.25 (m, 2H), 2.08 (q, 2H), 1.65 (d, 3H) ppm.

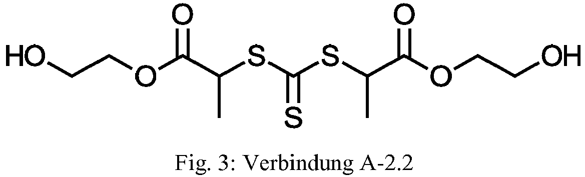

### Herstellung der telechelen Polyole (C):

### Beispiel 9: Umsetzung von Verbindung A-2.1 mit Styrol zu α-ω - Polystyrol-diol (C-1)

19,31 g A-2.1, erhältlich gemäß Bsp 6, 108,5 g Styrol, 521,8 g Dioxan sowie 2,58 g 2,2'-Azobis(2,4-dimethylvaleronitril) werden in einen Kolben vorgelegt und durch einen kontinuierlichen Stickstoffstrom für 30 min inertisiert. Anschließend wird für 22 Stunden auf 60 °C erhitzt und mit 230 U/min gerührt. Nach Reaktionsende werde alle flüchtigen Bestandteile am Rotationsverdampfer entfernt und das erhaltene Produkt bis zur Gewichtskonstanz bei Raumtemperatur im Vakuum getrocknet. Die gravimetrische Ausbeute betrug 87,97 g. Laut GPC betrug Mₙ 2200 g/mol und der PDI 1,21.

### Beispiel 10: Umsetzung von Verbindung (A-2.1) mit Acrylnitril zu α-ω) - Polyacrylnitrildiol (C-2)

3,0 g A-2.1 erhältlich gemäß Beispiel 6, 21,5 g Acrylnitril, 102,0 g Dimethylsulfoxid sowie 1,004 g 2,2'-Azobis(2,4-dimethylvaleronitril) werden in einen Kolben vorgelegt und durch einen kontinuierlichen Stickstoffstrom für 30 min inertisiert._Anschließend wird für 3 Stunden auf 60 °C erhitzt und mit 230 U/min gerührt. Nach Reaktionsende wird die Reaktionslösung in reichlich Wasser gegeben, das dabei ausgefällte Produkt wird abfiltriert und bis zur Gewichtskonstanz bei Raumtemperatur im Vakuum getrocknet. Die gravimetrische Ausbeute betrug 1,65 g. Laut GPC betrug Mₙ 3980 g/mol und der PDI 1,31.

### Beispiel 11: Umsetzung von Verbindung (A-2.1) mit Styrol und Acrylnitril zu α-ω-Polystyrol-block-polyacrylnitril-diol (C-3)

4,2 g A-2.1 erhältlich gemäß Besipiel 6, 3,18 g Styrol, 29,52 g Dioxan sowie 0,149 g 2,2'-Azobis(2,4-dimethylvaleronitril) werden in einen Kolben vorgelegt und durch einen kontinuierlichen Stickstoffstrom für 30 min inertisiert._Anschließend wird für 9 Stunden auf 60 °C erhitzt und mit 230 U/min gerührt. Anschließend werden 6,36 g Acrylnitril und 0,298 g 2,2'-Azobis(2,4-dimethylvaleronitril) gelöst in 32,0 g Dioxan zugegeben und für weitere 21 Stunden auf 60 °C erhitzt und mit 230 U/min gerührt. Nach Reaktionsende wird die Reaktionslösung mit 200 mL Methanol versetzt, das dabei ausfallende Produkt wird abfiltriert und bis zur Gewichtskonstanz bei Raumtemperatur im Vakuum getrocknet. Die gravimetrische Ausbeute betrug 3,82 g. Laut GPC betrug Mₙ 3180 g/mol und der PDI 1,62.

### Beispiel 12: Umsetzung von Verbindung (A-2.2) mit Butylacrylat zu α-ω - Polyacrylat-diol (C-4)

0,17 g A-2.2 erhältlich gemäß Beispiel 8, 3,2 g Butylacrylat, 13,6 g Chlorbenzol sowie 0,072 g 2,2'-Azobis(2-methyl-N-(2-hydroxyethyl)propionamid)_werden in einen Kolben vorgelegt und durch einen kontinuierlichen Stickstoffstrom für 30 min inertisiert. Anschließend wird für 7,5 Stunden auf 90 °C erhitzt und mit 130 U/min gerührt. Nach Reaktionsende werde alle flüchtigen Bestandteile am Rotationsverdampfer entfernt und das erhaltene Produkt bis zur Gewichtskonstanz bei Raumtemperatur im Vakuum getrocknet. Die gravimetrische Ausbeute betrug 2,5 g. Laut GPC betrug Mₙ 3330 g/mol und der PDI 1,25.

## Patentansprüche

1. H-funktionelles Trithiocarbonat (A) als Reagenz für die reversible Additions-Fragmentierungs-Kettenübertragungs-Polymerisation (RAFT), wobei das H-funktionelle Trithiocarbonat (A) die Struktur (A-1) 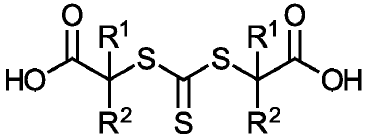 mit
R¹ ausgewählt wird aus der Gruppe bestehend aus Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
R² ausgewählt wird aus der Gruppe bestehend aus Wasserstoff, Methyl, Ethyl, Propyl , iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl Octyl, Phenyl, Benzyl und Cyclohexyl
und/oder Struktur (A-2) 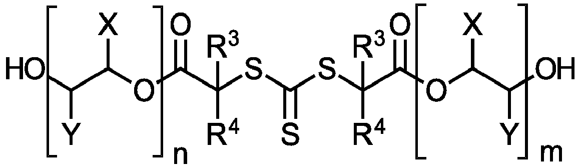 mit
n und m unabhängig voneinander 1 bis 100, bevorzugt 1 bis 50, besonders bevorzugt 1 bis 10 und ganz besonders bevorzugt 1 bis 4
R³ ausgewählt wird aus der Gruppe bestehend aus Methyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
R⁴ ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl Octyl, Phenyl, Benzyl und Cyclohexyl.
X und Y unabhängig voneinander ausgewählt wird aus der Gruppe bestehend aus Wasserstoff, Methyl, Ethyl, Propyl, Butyl, Phenyl, CH₂Cl, CH₂Br, CH₂OH, CH₂OCH₃, CH₂OCH₂CH₃, C(O)OCH₃, C(O)OCH₂CH₃, -(CH₂)₇CH₃, - (CH₂)₇C(O)OH, -(CH₂)₇C(O)OCH₃ und -(CH₂)₇C(O)OCH₂CH₃
und/oder Struktur (A-3) 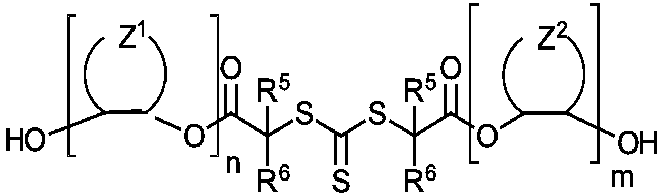 mit
n und m unabhängig voneinander 1 bis 100, bevorzugt 1 bis 50, besonders bevorzugt 1 bis 10 und ganz besonders bevorzugt 1 bis 4
R⁵ ausgewählt wird aus der Gruppe bestehend aus Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl und 3-Methylpentyl und
R⁶ ausgewählt wird aus der Gruppe bestehend aus Wasserstoff, Methyl, Ethyl, Propyl, iso-Propyl, Butyl, sec-Butyl, tert-Butyl, Pentyl, Hexyl, Heptyl, Octyl, Nonyl, Decyl, Cyclopentyl, Cyclohexyl, 1,1-Dimethylethyl, 3-Methylpentyl, CN, OH, COOH, NO₂, Cl, Br, I, SH, -NH₂, NHR⁷-, NR⁸R⁹- und C(O)OR¹⁰, wobei R⁷, R⁸, R⁹, R¹⁰ unabhängig voneinander ausgewählt sind aus der Gruppe bestehend aus Alkyl, Aryl, Cycloalkyl bevorzugt Methyl, Ethyl, Propyl, Butyl, Pentyl, Hexyl, Heptyl,Octyl, Phenyl, Benzyl undCyclohexyl
Z¹, Z² unabhängig voneinander ausgewählt wird aus der Gruppe bestehend aus - (CH₂)₄-, -(CH₂)₅-, -(CH₂)₆-, -(CH₂)₇-, -(CH₂)₈-, -(CH₂(SO₂)CH₂)-, - (CH₂CH₂CHCHCH₂)- und -((CH₂)₃CHCH(CH₂)₃)-.
aufweist.

2. H-funktionelles Trithiocarbonat (A) gemäß Anspruch 1, wobei das Trithiocarbonat (A) folgende die Struktur (A-1) oder (A-2) mit
R¹ = R³ = Methyl,
R² = R⁴ = Wasserstoff,
und
X und Y unabhängig voneinander ausgewählt wird aus der Gruppe bestehend aus Wasserstoff und Methyl, Ethyl, Propyl, Butyl, Phenyl, -CH₂Cl, -CH₂Br, -CH₂OH,-CH₂OCH₃, -CH₂OCH₂CH₃, -C(O)OCH₃, -C(O)OCH₂CH₃, -(CH₂)₇CH₃, -(CH₂)₇COOH, -(CH₂)₇COOCH₃, -(CH₂)₇COOCH₂CH₃, und -CH₂OCH₂CHCH₂.
aufweist.

3. H-funktionelles Trithiocarbonat (A) gemäß Anspruch 1 oder 2, wobei das Trithiocarbonat (A) die Struktur (A-2) aufweist und eine oder mehrere Verbindungen ausgewählt aus der Gruppe bestehend aus:
(A-2.1(1)) 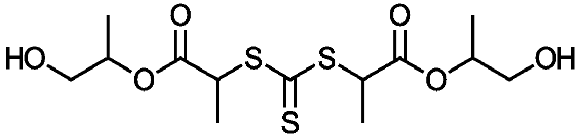
, (A-2.1(2)) 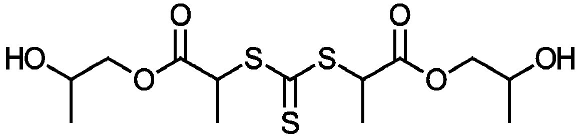
, (A-2.1(3)) 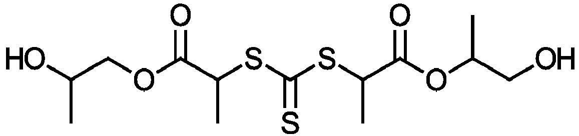 oder
und (A-2.2)

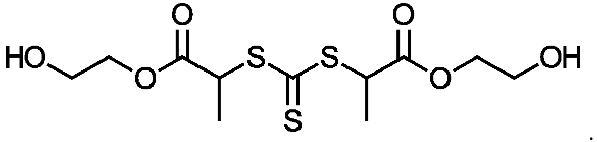

4. Verfahren zur Herstellung eines H-funktionelles Trithiocarbonat (A) umfassend folgende Schritte:
i-1) Bereitstellung von einem oder mehreren Trithiocarbonat-Salz(en)
i-2) Umsetzung des Trithiocarbonat-Salzes aus Schritt i-1) mit einer aliphatischen, Halogen-haltigen H-funktionelle Verbindung zum H-funktionelle Trithiocarbonat (A) mit der Struktur (A-1).
i-3) Umsetzung der in Schritt i-2) erhaltenden Verbindung mit der Struktur (A-1) mit einem Alkylenoxid in Gegenwart eines Katalysators und eines Lösungsmittels zu einer Verbindung mit der Struktur (A-2),
wobei der Katalysator in Schritt i-3) eine Chrom(III)-haltige Verbindung ist und das in Schritt i-3) verwendete Lösungsmittel ein aprotisches Lösungsmittel ist.

5. Verfahren gemäß Anspruch 4, wobei das Trithiocarbonat-Salz in Schritt i-1) durch basenkatalysierte Umsetzung von Schwefelkohlenstoff erhalten wird.

6. Verfahren gemäß Anspruch 4 oder 5, wobei die Halogen-haltige H-funktionelle Verbindung eine oder mehrere Verbindung(en) ist und ausgewählt wird aus der Gruppe bestehend aus 2-Brompropionsäure, 2-Brombutansäure, 2-Brompentansäure, 2-Bromhexansäure, 3-Brombutansäure, 3-Brompentansäure, 3-Bromhexansäure, 2-Brom-2-methlypropionsäure, 2-Brom-2-methlybutansäure, 7-Bromölsäure, 8-Bromölsäure, 2-Brompropanol, 2-Brombutanol, 2-Brompentanol, 2-Bromhexanol, Bromcyclohexanol, 2-Bromheptanol, 2-Bromoctanol, 3-Brombutanol, 3-Brompentanol, 3-Bromhexanol, 3-Bromheptanol, 3-Bromoctanol, 2-Chlorpropionsäure, 2-Chlorbutansäure, 3-Chlorbutansäure, 2-Iodpropionsäure, 2-Iodbutansäure, 3-Iodbutansäure, 2-Chlorpropanol, 2-Chlorbutanol, 3-Chlorbutanol, 2-Iodpropanol, 2-Iodbutanol und 3-Iodbutanol.

7. Verfahren gemäß einem der Anspruch 4 bis 6, wobei das Alkylenoxid in Schritt i-3) eine oder mehrere Verbindung(en) ist und ausgewählt wird aus der Gruppe bestehend aus Ethylenoxid, Propylenoxid, Epichlorhydrin, Epibromhydrin, Glycidol, Cyclohexenoxid, 9,10-Epoxystearinsäure, 9,10-Epoxyoctadecan-1-ol, Allylglycidylether, 2,3-Epoxybutan, und Styroloxid bevorzugt Ethylenoxid und Propylenoxid.

8. Verfahren gemäß einem der Ansprüche 4 bis 7, wobei die Reaktionstemperatur T₂ in Schritt i-3) von 50 °C bis 200 °C, bevorzugt von 80 °C bis 150 °C, besonders bevorzugt von 90 °C bis 130 °C beträgt.

9. Verfahren gemäß Anspruch 8, wobei die als Katalysator in Schritt i-3) verwendete Chrom(III)-haltige Verbindung eine oder mehrere Verbindung(en) ist ausgewählt wird aus der Gruppe bestehend aus Chromfluorid, Chromchlorid, Chrombromid, Chromiodid, Chromoxid, Chromacetylacetonat, Chromacetat, Chromformiat, Chromoxalat, Chromnitrat, Chromcarbonat, Chromsulfate und Chrompicolinat bevorzugt Chromchlorid.

10. Verfahren gemäß einem der Ansprüche 4 bis 9, wobei das in Schritt i-3) verwendete Lösungsmittel ein aprotisch-unpolares Lösungsmittel ist.

11. Verfahren gemäß Anspruch 10, wobei das aprotisch-unpolare Lösungsmittel Lösungsmittel eine oder mehrere Verbindung(en) ist und ausgewählt wird aus der Gruppe bestehend aus Methylphenylether, Tetrahydrofuran, 2-Methyltetrahydrofuran, 2-Methoxy-2-methylpropan, Butanon, Aceton, Toluol, Benzol, Xylol, n-Pentan, n-Hexan, n-Heptan, Cyclopentan, Cyclohexan, Cycloheptan, Ethylacetat, *iso*-Propylacetat, *n-*Butylacetat Diethylether, Methylpropylether, sec-Butylmethylether, Dimethylacetal, Ethoxymethoxymethan, Dimethylether, Di-iso-propylether, 2,2-Dimethoxypropane, Chlorbenzol, Dichlorbenzol, Dichloromethan, Chloroform, 1,2-Dichlorethan, und 1,1,2,2-Tetrachlorethan, bevorzugt Methylphenylether, Ethylacetat, Toluol, Chlorbenzol, Dichlorbenzol und Xylol.

12. Verfahren zur Herstellung eines telechelen Polyols (C), bevorzugt telechelen Diols, umfassend der Umsetzung eines H-funktionellen Trithiocarbonats (A) mit der Struktur (A-1) oder (A-2) oder (A-3) gemäß einem der Ansprüche 1 bis 3 oder ein H-funktionelles Trithiocarbonat (A) erhältlich nach einem der Ansprüche 4 bis 11 mit einem doppelbindungshaltigen Monomer (B) in Gegenwart eines Radikalstarters, wobei das doppelbindungshaltige Monomer (B) mit der folgenden Struktur aufweist: 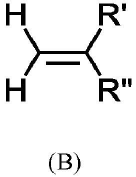 , wobei
R'= H, C1-C4 Alkylgruppen
R"= Halogen, Hydroxy-substituierte Arylgruppen, Hydroxy-unsubstituierte Arylgruppen, Halogen-substituierte C1-C10 Alkylgruppen, Hydroxy-substituierte C1-C10 Alkylgruppen, Alkylsäureestergruppen.

13. Verfahren gemäß Anspruch 12, wobei das doppelbindungshaltigen Monomer (B) eine oder mehrere Verbindung(en) ist und ausgewählt wird aus der Gruppe bestehend aus Vinylchlorid, Styrol, Butylacrylat, Acrylnitril, 2-Ethylhexylacrylat, Methylacrylat, Methylmethacrylat, Ethylacrylat, Ethylmethacrylat, Propylacrylat, Propylmethacrylat, Butylacrylat, Isobutylacrylat, Butylmethacrylat, Cyclohexylmethacrylat, (2-Ethylhexyl)acrylate, Isobornylmethacrylat, (Hydroxyethyl)methacrylat, N-Vinyl-pyrrolidon, N-Vinyl-pyrrolidon, N-Vinyl-caprolactam, N-Vinyl-caprolactam, N-Vinyl-imidazol, N-Vinyl-imidazol, N-Vinyl-N-Methylacetamid, Ethylvinylether, n-Butylvinylether, *iso*-Butylvinylether, Cyclohexylvinylether, 2-Ethylhexylvinylether, Dodecylvinylether, Octadecylvinylether, 1,4-Butanedioldivinylether, Diethyleneglycoldivinylether, Triethyleneglycoldivinylether , 1,4-Cyclohexanedimethanoldivinylether, Hydroxybutylvinylether, 1,4-Cyclohexanedimethanolmonovinylether, 1,2,4-Trivinylcyclohexane, 3-Aminopropylvinylether bevorzugt Styrol, Acrylnitril, Vinylacetat, Butylacrylat, Vinylchlorid, Methylacrylat, Ethylacrylat und Methylmethacrylat.

14. Telecheles Polyol (C), bevorzugt telecheles Diol herstellbar nach einem Verfahren gemäß Anspruch 12 oder 13.

15. Verfahren zur Herstellung eines Trithiocarbonat-freien, telechelen Polyols (D), bevorzugt telechelen Diols, durch Umsetzung des telechelen Polyols (C), bevorzugt telecheles Diol, gemäß Anspruch 14 mit einem Oxidationsmittel.
